# EUROPÄISCHE PATENTSCHRIFT

(11) **EP 2 166 005 B1**
(45) Veröffentlichungstag und Bekanntmachung des Hinweises auf die Patenterteilung: **29.08.2012**
(21) Anmeldenummer: 09164707.3
(22) Anmeldetag: 07.07.2009
(51) Int. Cl.: C07D 263/10

(54) **Verfahren zur Herstellung von Phenylenbisoxazolinen**
Method for making phenylenebisoxazolines
Procédé de fabrication des phenylènebisoxazolines

(30) Priorität: 23.09.2008 EP 08164919
(43) Veröffentlichungstag der Anmeldung: 24.03.2010
(73) Patentinhaber: Evonik Degussa GmbH, 45128 Essen (DE)
(72) Erfinder: Kuebelbaeck, Thomas, 48249, Dülmen (DE); Neumann, Manfred, 45770, Marl (DE); Omeis, Marianne, 46286, Dorsten (DE)

(56) Entgegenhaltungen:
- EP-A- 1 548 012
- DE-A1- 2 135 644
- US-A- 4 806 267

## Beschreibung

Die vorliegende Erfindung beschreibt ein Verfahren zur Herstellung von Heterocyclen der Formel (I) durch katalytische Umsetzung eines aromatischen Dinitrils und einem Aminoalkohol.

Die durch das erfindungsgemäße Verfahren zugänglichen Heterocyclen der Formel (I) können u.a. als Kettenverlängerer oder Vernetzer, in Polymeren eingesetzt werden (US 4,806,267, Culbertson in Prog. Polym. Sci. 27 (2002) 579-626).

Die Offenlegungsschrift DE 21 35 644 A1 beschreibt ein nicht-katalytisches Verfahren zur Herstellung von cyclischer Imidsäureester aus aromatischen Nitrilen und Aminoalkanolen, das eine Reaktionszeit von über 20 Stunden aufweist. Die Umsetzung erfolgt hierbei unter Schutzgas, um die Nebenproduktbildung zurückzudrängen. Die Aufarbeitung des Reaktionsgemisches erfolgt u.a. durch Umkristallisation.

Ebenfalls ein Verfahren zur Herstellung von Oxazinen beschreibt die DE 21 53 513 A1 jedoch durch Umsetzung von Dicarbonsäure-bis-(3-halogen-propylamiden) oder Dicarbonsäure-bis-(3-halogenpropylimidoestern) in Gegenwart einer Base. Auch hier erfolgt die Aufarbeitung durch Umkristallisation.

Culbertson et al. beschreiben in der US 4,806,267 ein Verfahren zur Herstellung solcher Bisoxazinen -jedoch in Form von Mischungen mit Bisoxazolinen - durch die Umsetzung von Mischungen verschiedener Aminoalkanolen mit Dinitrilen in Gegenwart von Cadmiumnitrat bzw. Zinkacetat als Katalysator und Xylol als Lösemittel. Die Reaktionszeit beträgt auch hier 10 bzw. 20 Stunden.

In Prog. Polym. Sci. 27 (2002) 579-626 beschreibt Culbertson ebenfalls die Synthese von Oxazinen durch die Umsetzung von Nitrilen und Aminoalkoholen in Gegenwart von Zinkacetat als Katalysator und Xylol als Lösemittel.

Die EP 1 548 012 A2 beschreibt ein katalytisches Verfahren zur Herstellung von Phenylenbisoxazolinen durch Umsetzung von Terephthalodinitril bzw. Isophthalodinitril mit 1,2-Aminoalkoholen in Gegenwart von zinkhaltigen Katalysatoren und Xylol als Lösemittel. In einer weiteren Verfahrensvariante erfolgt die Umsetzung in Abwesenheit eines zusätzlichen Lösemittels, wie beispielsweise Xylol.

Es war die Aufgabe der vorliegenden Erfindung, ein im technischen Maßstab durchführbares Verfahren bereitzustellen, das sich durch eine verbesserte Raum-Zeit-Ausbeute auszeichnet und ferner die Abtrennung des Zielproduktes aus dem Reaktionsgemisch beim Einsatz der höheren Homologen des Aminoalkohols ermöglicht. Insbesondere sollte das erfindungsgemäße Verfahren das Rezyklieren der überschüssigen Reagenzien bzw. der Lösemittel ermöglichen.

Überraschenderweise wurde ein Verfahren zur Herstellung von Heterocyclen der Formel (I) - insbesondere von Oxazinen - gefunden, das sich durch eine verbesserte Raum-Zeit-Ausbeute auszeichnet. Das erfindungsgemäße Verfahren wird ohne den Zusatz eines zusätzlichen Lösemittels während der Reaktion durchgeführt, wobei das Edukt Aminoalkohol der Formel (III) im Überschuss der Reaktionsmischung zugegeben wird und somit als Lösemittel wirken kann. Die Reaktionsmischung kann bei den Verfahrensbedingungen gut gerührt werden. Ferner sind die Reaktionszeiten mit unter 10 Stunden relativ kurz und auch die Ausbeuten des erfindungsgemäßen Verfahrens sind mit mindestens 75 % gut.

Die Aufarbeitung der Reaktionsmischung beim Einsatz der höheren Homologen der Aminoalkohole ist im Gegensatz zu den Verfahren zur Herstellung von Phenylenbisoxazolinen äußerst schwierig. Die Reaktionsmischung wird häufig zum Ende der Reaktion gelartig, so dass eine Abtrennung, beispielsweise durch Filtration, nicht möglich ist. In anderen Fällen bildet das gewünschte Zielprodukt einen sehr fein verteilten Niederschlag in der Reaktionsmischung, der sich nur über einen sehr langen Zeitraum abfiltrieren lässt - mit der Gefahr, dass der Filter sich zusetzt.

Überraschenderweise kann diese Aufarbeitung der Reaktionsmischung beim Einsatz der höheren Homologen des Aminoalkohols deutlich vereinfacht werden, indem der Aminoalkohol der Formel (III) restlos bzw. nahezu vollständig durch ein geeignetes unpolares Lösemittel ersetzt wird. Auf diese Weise erhält man ein Rohprodukt mit einem Restgehalt an Aminoalkohol der Formel (III) von maximal 20 Gew.-% bezogen auf die Menge an Heterocyclen der Formel (I). Aus diesem unpolaren Lösemittel kann nun das gewünschte Zielprodukt auskristallisieren, welches sich nun einfach mittels Filtration abtrennen lässt. Entscheidend für die Kristallisation bzw. Filtration der Heterocyclen der Formel (I) ist die Abwesenheit des Aminoalkohols der Formel (III) bzw. das Vorliegen dieses Aminoalkohols in sehr geringer Konzentration im Rohprodukt, da im Gegensatz zu den Phenylenbisoxazolinen bei den höheren Homologen des Aminoalkohols keine ausreichende Kristallbildung in Gegenwart des entsprechenden Aminoalkohols erfolgt.

Gegenstand der Erfindung ist somit ein Verfahren gemäß Anspruch 1 zur Herstellung von Heterocyclen der Formel (I) 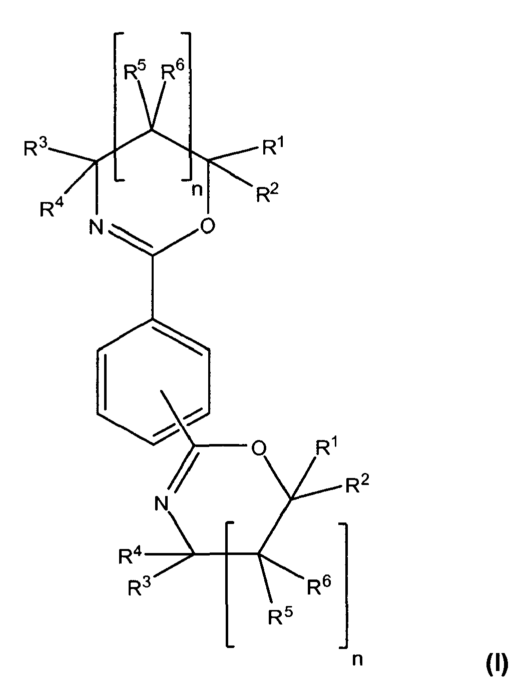 durch katalytische Umsetzung eines aromatischen Dinitrils der Formel (II) 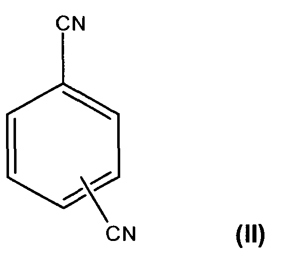 mit einem Aminoalkohol der Formel (III) 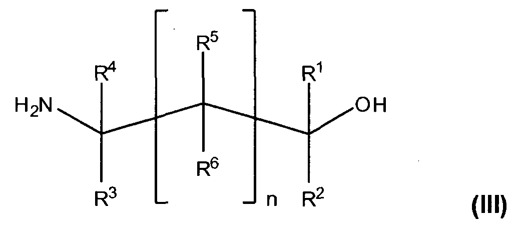 mit R¹, R², R³, R⁴, R⁵ und R⁶ gleich oder voneinander verschieden sind und jeweils Wasserstoff, Alkyl, Aryl, -COOH oder -NH₂ bedeuten und n = 0 bis 4, vorzugsweise n = 1 bis 4, bevorzugt n = 1 bis 3 und besonders bevorzugt n = 1 bis 2 bedeutet, das sich dadurch auszeichnet, dass der Aminoalkohol der Formel (III) und der Katalysator vorgelegt und das aromatische Dinitril der Formel (II) bei Reaktionstemperatur zudosiert wird, wobei auf ein zusätzliches Lösemittel während der Reaktion verzichtet wird, und anschließend nach dem Reaktionsende der überschüssige Aminoalkohol vollständig bzw. nahezu vollständig durch ein unpolares Lösemittel ersetzt wird, und ein Rohprodukt mit einem Gehalt an Aminoalkohol von maximal 20 Gew.-% bezogen auf die Menge an Heterocyclen der Formel (I) erhalten wird.

Als Aminoalkohol der Formel (III) werden in dem erfindungsgemäßen Verfahren vorzugsweise Verbindungen mit n = 1 bis 4, bevorzugt mit n = 1 bis 3 und besonders bevorzugt n = 1 bis 2 eingesetzt. Bevorzugt weisen die Aminoalkohole der Formel (III) als Substituenten vom Typ R¹, R², R³, R⁴, R⁵ und R⁶ Wasserstoff oder verzweigte bzw. unverzweigte Alkylgruppen mit 1 bis 4 Kohlenstoffatomen auf, besonders bevorzugt sind alle der Substituenten vom Typ R¹, R², R³, R⁴, R⁵ und R⁶ Wasserstoff. Ganz besonders bevorzugt wird in dem erfindungsgemäßen Verfahren 3-Amino-1-propanol eingesetzt.

In dem erfindungsgemäßen Verfahren können auch Mischungen an verschiedenen Aminoalkoholen der Formel (III) eingesetzt werden. Da der Aminoalkohol sowohl als Edukt als auch als Lösemittel während der Reaktion dient, ist es vorteilhaft nur ein Aminoalkohol der Formel (III) und keine Mischungen an Aminoalkoholen einzusetzen. Auf diese Weise können mittels des erfindungsgemäßen Verfahrens Heterocyclen der Formel (I) gezielt hergestellt werden.

Als vorteilhaft hat sich bei dem erfindungsgemäßen Verfahren der Einsatz von Zinkverbindungen als Katalysator erwiesen, wobei sowohl eine Zinkverbindung als auch ein Gemisch von mehreren Zinkverbindungen nebeneinander eingesetzt werden können. Vorzugsweise werden dabei Zinkcarbonsäuresalze von gesättigten, aliphatischen Carbonsäuren mit zwei bis zehn Kohlenstoffatomen eingesetzt, die sowohl verzweigt als auch unverzweigt sein können, wie beispielsweise Zinkacetat, Zinkpropionat, Zink-n-butyrat, Zinkisobutyrat etc. oder auch Zink-2-ethylhexanoat, die jeweils als Einzelkomponente oder als Gemisch von mehreren Komponenten eingesetzt werden können.

Ganz besonders bevorzugt wird in dem erfindungsgemäßen Verfahren jedoch Zink-2-ethylhexanoat als Katalysator eingesetzt. Vorteilhaft beim Einsatz von Zink-2-ethylhexanoat ist, dass dieser flüssige, im Reaktionssystem relativ gut lösliche Katalysator nach der Filtration in der Mutterlauge verbleibt und somit ohne eine zusätzliche wässrige Aufarbeitung einfach und vollständig bzw. nahezu vollständig abgetrennt werden kann. Andere Zinkverbindungen, wie beispielsweise Zinkacetat, sind in der Reaktionsmischung des erfindungsgemäßen Verfahrens schwer löslich, so dass bei deren Verwendung ein zusätzlicher Verfahrensschritt in der Aufarbeitung der Reaktionsmischung notwendig ist, um den Katalysator vom Zielprodukt abzutrennen.

Die vorzugsweise zu verwendenden Zinkcarbonsäuresalze bieten den Vorteil großer katalytischer Aktivität bei gleichzeitig geringst möglicher Toxizität und einfacher Handhabbarkeit, bei gleichzeitig guter Verfügbarkeit.

In dem erfindungsgemäßen Verfahren wird der Aminoalkohol der Formel (III) und der Katalysator im Reaktor vorgelegt und das aromatische Dinitril der Formel (II) bei Reaktionstemperatur zudosiert. Die Zugabe des Dinitrils der Formel (II) sollte kontinuierlich über einen längeren Zeitraum, vorzugsweise über einen Zeitraum von 1 bis 4 Stunden, bevorzugt von 2 bis 3 Stunden, erfolgen. Dadurch bleibt die Konzentration an dem aromatischen Dinitril der Formel (II) im Reaktionsgemisch während der gesamten Reaktion niedrig und die Gasentwicklung lässt sich gut in einer technischen Anlage steuern.

Bei dem erfindungsgemäßen Verfahren kann das aromatische Dinitril der Formel (II) als Feststoff oder als Schmelze der Reaktionsmischung - bestehend aus Aminoalkohol der Formel (III) und Katalysator - zugegeben werden. Als aromatische Dinitril der Formel (II) werden in dem erfindungsgemäßen Verfahren vorzugsweise sowohl 1,3-Dinitril als auch 1,4-Dinitril eingesetzt. Es kann in dem erfindungsgemäßen Verfahren auch eine Mischung aus 1,3- und 1,4-Dinitril eingesetzt werden.

Das molare Verhältnis von Dinitril der Formel (II) zu Aminoalkohol (III) beträgt bei dem erfindungsgemäßen Verfahren mindestens 1 : 2, bevorzugt wird jedoch ein molares Verhältnis von 1 : (4 bis 10), besonders bevorzugt von 1 : (7 bis 9) angewendet.

Dieser stöchiometrische Überschuss an Aminoalkohol der Formel (III) ermöglicht den Verzicht auf ein zusätzliches Lösemittel, wie beispielsweise Xylol während der Reaktion. Die Reaktion wird somit in dem erfindungsgemäßen Verfahren ohne die Zugabe eines zusätzlichen Lösemittels durchgeführt. Als Lösemittel dient bei der Reaktion in diesem erfindungsgemäßen Verfahren der Aminoalkohol der Formel (III). Dadurch kann die Reaktion bei einer höheren Reaktionstemperatur durchgeführt werden. Von Vorteil ist hierbei insbesondere, dass das Reaktionsgemisch nicht siedet, wenn die Reaktionstemperatur unterhalb der Siedetemperatur des Aminoalkohols der Formel (III) liegt. Bei den Verfahren gemäß des Stands der Technik wird die Reaktion häufig in Gegenwart von Xylol durchgeführt. Xylol und einige Aminoalkohole der Formel (III) bilden Azeotrope mit einem Siedepunktsminimum, so dass die Reaktionsgemisch während der Umsetzung als auch während der Zugabe des Dinitrils siedet. Diese Reaktionsbedingungen beschränken zum einen die maximale Reaktionstemperatur zum anderen erfordern sie technische Lösungen für die Zugabe des Feststoffs Dinitril.

Die Reaktion kann daher erfindungsgemäß in einem Temperaturbereich von etwa 50 °C bis etwa 200 °C, vorzugsweise bei 110 °C bis 170 °C, bevorzugt aber in einem Temperaturbereich von 130 °C bis 150 °C durchgeführt werden.

Durch den Verzicht auf ein zusätzliches Lösemittel kann die Reaktionszeit verkürzt und somit die Raum-Zeit-Ausbeute deutlich verbessert werden. Ferner vereinfacht diese Vorgehensweise die Aufarbeitung der Reaktionsmischung.

Die Reaktion in dem erfindungsgemäßen Verfahren wird vorzugsweise bei einem Druck von 0,5 bar bis 10 bar, bevorzugt bei 0,6 bar bis 1,5 bar, besonders bevorzugt von 0,7 bis 1,2 bar und insbesondere bei Atmosphärendruck durchgeführt. In einer besonderen Ausführungsform des erfindungsgemäßen Verfahrens wird die Reaktion bei einem geringen Unterdruck durchgeführt, also bei einem Druck von 0,6 bis 0,9 bar, bevorzugt von 0,7 bis 0,9 bar. Dieser geringe Unterdruck bezogen auf den Atmosphärendruck ermöglicht eine einfachere Entfernung des während der Reaktion entstehenden Abgases Ammoniak.

Die erfindungsgemäße Umsetzung des Dinitrils der Formel (II) mit Aminoalkohol der Formel (III) kann sowohl diskontinuierlich, semi-kontinuierlich oder aber auch kontinuierlich durchgeführt werden. Dies kann je nach technischen Voraussetzungen flexibel gehandhabt werden, was einen weiteren wichtigen Vorteil des Verfahrens darstellt.

Bei dem erfindungsgemäßen Verfahren ist es von Vorteil nach der Zugabe des Dinitrils der Formel (II) das Reaktionsgemisch noch für 1 bis 5 Stunden, bevorzugt für 2 bis 4 Stunden, bei der Reaktionstemperatur zu rühren. Die exakte Dauer der Nachreaktionszeit kann durch kontinuierliche Analytik, beispielsweise mittels Gaschromatographie, des Abgasstroms ermittelt werden, die Nachreaktion kann beendet werden, sobald kein Ammoniak mehr detektiert werden kann.

Die Gesamtreaktionszeit des erfindungsgemäßen Verfahrens, die Zeit für die Zugabe der Reagenzien als auch die Nachreaktion, ist dabei abhängig von der Temperatur und kann erfindungsgemäß von 2 bis 9 Stunden reichen. Bei Reaktionstemperaturen von 110 °C bis 170 °C werden jedoch vorzugsweise Gesamtreaktionszeiten von 4 bis 88 Stunden, insbesondere von 5 bis 7 Stunden angewendet. Diese relativ kurze Reaktionszeit bedeutet einen weiteren Vorteil gegenüber den bisher bekannten Verfahren, bei denen teilweise deutlich längere Reaktionszeiten von 20 bis 25 Stunden angewendet werden müssen. Dadurch gelingt es die Raum-Zeit-Ausbeuten in vorteilhafter Weise zu erhöhen, was für die Wirtschaftlichkeit eines technischen Verfahrens eine ganz bedeutende Rolle spielt.

Im Anschluss an die Nachreaktion des erfindungsgemäßen Verfahrens erfolgt die Abtrennung des überschüssigen Aminoalkohols, vorzugsweise mittels einer Destillation.

So wird in einer bevorzugten Ausführungsform der Aufarbeitung des Reaktionsgemisches des erfindungsgemäßen Verfahrens zunächst 40 bis 70 Gew.-%, besonders bevorzugt 55 bis 65 Gew.-% des Überschusses an Aminoalkohol der Formel (III) bei einem reduzierten Druck, besonders bevorzugt bei einem Druck von 75 bis 400 mbar und ganz besonders bevorzugt bei 100 bis 300 mbar, destillativ entfernt.

Anschließend wird vorzugsweise der in dem Reaktionsgemisch noch nach der destillativen Abtrennung des Aminoalkohols verbliebene Anteil des Aminoalkohols der Formel (III) unter Zugabe eines zusätzlichen unpolaren Lösemittels abdestilliert, wobei das zusätzliche unpolare Lösemittel bevorzugt kontinuierlich zudosiert wird, während gleichzeitig der Aminoalkohol zusammen mit dem zusätzlichen unpolaren Lösemittel aus dem Reaktionsgemisch destillativ entfernt wird. Besonders bevorzugt wird hierbei soviel zusätzliches unpolares Lösemittel dem Reaktionsgemisch zudosiert, wie an Destillat, weitgehend bestehend aus Aminoalkohol und unpolaren Lösemittel, abdestilliert wird Das zusätzliche unpolare Lösemittel dient nun an Stelle des Aminoalkohols der Formel (III) als Lösemittel und/oder Suspensionsmittel für das Zielprodukt - dem Heterocyclus der Formel (I). Der abdestillierte Aminoalkohol als auch das abdestillierte zusätzliche unpolare Lösemittel können dem erfindungsgemäßen Verfahren an den entsprechenden Stellen zurückgeführt werden.

Als zusätzliches unpolares Lösemittel eignen sich Lösemittel, die einen Siedepunkt von mindestens 100 °C, bevorzugt von 115 °C bis 145 °C aufweisen und inert gegenüber dem Aminoalkohol der Formel (III) und dem Heterocyclus der Formel (I) sind. Insbesondere werden in dem erfindungsgemäßen Verfahren aromatische Lösemittel, wie beispielsweise Toluol, Ethylbenzol, o-, m- oder p-Xylol, und Mischung dieser aromatischen Lösemittel eingesetzt. Bevorzugt werden unpolare Lösemittel eingesetzt, die zusammen mit dem jeweils eingesetzten Aminoalkohol der Formel (III) ein Azeotrop mit einem Siedepunktsminimum bilden. Beim Einsatz von 3-Amino-1-propanol eignet sich hierfür als unpolares Lösemittel Xylol.

Auf diese Weise erhält man ein Rohprodukt, das einen Gehalt an Aminoalkohol der Formel (III) von maximal von maximal 20 Gew.-%, bevorzugt von 3 bis 10 Gew.-%, bezogen auf die Menge an Heterocyclen der Formel (I) aufweist, aus dem sich nun die Heterocyclen der Formel (I) gut auskristallisieren lassen. Das Rohprodukt weist somit vorzugsweise als Lösemittel das unpolare Lösemittel mit einem maximalen Gehalt an Aminoalkohol der Formel (III) von 20 Gew.-%, bevorzugt von 10 Gew.-%, bezogen auf die Menge an Heterocyclen der Formel (I) auf.

Aufgrund des sehr geringen Gehalts an Aminoalkohol der Formel (III) im Rohprodukt bildet sich im Gegensatz zu dem Verfahren gemäß EP 1 548 012 A2 keine zwei Flüssigphasen aus, so dass auf den Einsatz eines Lösungsvermittlers, wie beispielsweise Alkohole, die von dem eingesetzten Aminoalkohol der Formel (III) verschieden sind, verzichtet werden kann. Dieser Verzicht auf Lösungsvermittler hat den Vorteil, dass die Aufarbeitung des überschüssigen Aminoalkohols der Formel (III) und des unpolaren Lösemittels sich vergleichsweise einfach gestaltet, insbesondere da gerade das iso-Propanol - wie es in der EP 1 548 012 A2 eingesetzt wird - mit einigen Aminoalkoholen der Formel (III) ein Azeotrop ausbilden.

Aus diesem Rohprodukt kann nun das gewünschte Zielprodukt die Heterocyclen der Formel (I) auskristallisieren. Auf diese Weise enthält man Kristalle der Heterocyclen der Formel (I), die sich - im Gegensatz zu Verfahren gemäß dem Stand der Technik - mit dem Fachmann bekannten Methoden einfach abfiltrieren lassen. Vor allem ermöglicht diese erfindungsgemäßen Verfahren ein Abfiltrieren der Heterocyclen der Formel (I) ohne ein Verkleben der Filter. Aufgrund der Luftempfindlichkeit der Heterocyclen der Formel (I) wird die Filtration bevorzugt in einer Stickstoffatmosphäre durchgeführt.

Die nach der Filtration zurückbleibende Mutterlauge kann in den Verfahrensschritt der Aufarbeitung zurückgeführt werden, vorzugsweise erfolgt dies vor der gemeinsamen Destillation von Aminoalkohol und unpolaren Lösemittel gemeinsam mit oder anstelle des unpolaren Lösemittels. Durch diese Rückführung der Mutterlauge kann die Ausbeute noch weiter gesteigert werden. Die Rückführung der Mutterlauge ist in dem erfindungsgemäßen Verfahren deutlich gegenüber der EP 1 548 012 A2 vereinfacht, da die Mutterlauge überwiegend aus dem unpolaren Lösemittel besteht. Um die Mutterlauge in dem erfindungsgemäßen Verfahren zu rezyklieren, ist daher keine aufwändige Aufarbeitung notwendig, insbesondere da auf einen Lösungsvermittler vorzugsweise verzichtet wird.

Wird bei der Reaktion des erfindungsgemäßen Verfahrens als Katalysator Zink-2-ethylhexanoat eingesetzt, so verbleibt der Katalysator in der Mutterlauge. Ein weiterer Aufarbeitungsschritt, um den Katalysator von dem gewünschten Zielprodukt abzutrennen, ist somit nicht notwendig. Auf diese Weise lässt sich zum einen der Katalysator sehr einfach abtrennen und zum anderen erhält man Kristalle mit einer hohen Reinheit.

Die Kristalle der Heterocyclen der Formel (I) werden in dem erfindungsgemäßen Verfahren vorzugsweise mit einem Alkohol mit 1 bis 4 Kohlenstoffatomen, bevorzugt Methanol, Ethanol oder iso-Propanol, besonders bevorzugt Methanol, mehrmals gewaschen und unter Vakuum, bevorzugt bei einem Druck von 10 bis 25 mbar und einer Temperatur von 80 °C bis 100 °C für 3 bis 5 Stunden getrocknet.

Wurde bei der Reaktion des erfindungsgemäßen Verfahren eine schwerlösliche Zinkverbindung als Katalysator eingesetzt, wie beispielsweise Zinkacetat, so werden die Kristalle der Heterocyclen der Formel (I) vorzugsweise zunächst mit Wasser gewaschen, um den Katalysator abzutrennen. Erst anschließend werden dann die Kristalle mit dem Alkohol gewaschen.

Die nachfolgenden Beispiele sollen das erfindungsgemäße Verfahren näher erläutern, ohne dass die Erfindung auf diese Ausführungsform beschränkt sein soll.

### Beispiel 1:

Die Reaktion wird in einem 2 Liter-4-Halsrührkolben mit Innenthermometer, Flügelrührer mit Rührhülse und Rührmotor, Beheizung mittels Ölbad, Wasserauskreiser, Rückflusskühler und einer Abgasleitung mit einem Blasenzähler zur Überwachung des frei werden Ammoniaks durchgeführt. Hierfür werden 600,0 g 3-Amino-1-propanol (7,9 mol) und 5,0 g Zink-2-ethlyhexanoat (0,01 mol) im Reaktionsgefäß vorgelegt und unter Rühren auf eine Temperatur von ca. 140 °C eingestellt. Kontinuierlich werden 128,0 g Terephthalsäuredinitril (1,0 mol) in 2,5 Stunden zu der Reaktionslösung in den Reaktor gegeben. Nach Ende der Terephthalsäuredinitril-Zugabe wird 3,5 Stunden bei 140 °C nachgerührt. Im Reaktor liegt eine Lösung vor. Der Reaktionsverlauf wird sowohl über die Abgasbildung als auch über die GC-Analytik bestimmt. Am Ende der Nachreaktionszeit entsteht nur noch minimal Abgas. Die Reaktion wird bis zu einem Umsatz von > 99 % Richtung Zielprodukt durchgeführt.

Nach Beendigung der Reaktion wird ein Teil (ca. 280 g) des überschüssigen 3-Amino-1-propanol bei reduziertem Druck (ca. 150 mbar) destillativ entfernt. Das verbleibende 3-Amino-1-propanol wird durch Zugabe von 325 g Xylol zu dem Reaktionsgemisch durch eine gemeinsame Destillation von Xylol und 3-Amino-1-propanol nahezu vollständig entfernt.

Anschließend lässt man das Reaktionsgemisch unter Rühren und einer Stickstoffatmosphäre auf 20 °C abkühlen. Die sich gebildeten Kristalle des Heterocyclus werden über eine Glasfilternutsche abgesaugt, nacheinander dreimal mit Methanol auf der Nutsche aufgeschlämmt und trocken gesaugt.

Die feuchten Kristalle werden in einem Vakuumtrockenschrank bei 25 bis 10 mbar und 80 °C bis 100 °C 4 Stunden lang getrocknet. Man erhält das Produkt als reinweiße, rieselfähige Kristalle (GC- und NMR- Reinheit: > 99 %; Schmelzbereich: 216 °C bis 220 °C). Die Ausbeute dem Heterocyclus gemäß Formel (I) beträgt 75%.

### Vergleichsbeispiel 1:

Die Reaktion wird in einem 2 Liter-4-Halsrührkolben mit Innenthermometer, Flügelrührer mit Rührhülse und Rührmotor, Beheizung mittels Ölbad, Wasserauskreiser, Rückflusskühler und einer Abgasleitung mit einem Blasenzähler zur Überwachung des frei werden Ammoniaks durchgeführt. Hierfür werden 600,0 g 3-Amino-1-propanol (7,9 mol) und 5,0 g Zink-2-ethlyhexanoat (0,01 mol) im Reaktionsgefäß vorgelegt und unter Rühren auf eine Temperatur von ca. 140 °C eingestellt. Kontinuierlich werden 128,0 g Terephthalsäuredinitril (1,0 mol) in 2,5 Stunden zu der Reaktionslösung in den Reaktor gegeben. Nach Ende der Terephthalsäuredinitril-Zugabe wird 3,5 Stunden bei 140 °C nachgerührt. Im Reaktor liegt eine Lösung vor. Der Reaktionsverlauf wird sowohl über die Abgasbildung als auch über die GC-Analytik bestimmt. Am Ende der Nachreaktionszeit entsteht nur noch minimal Abgas. Die Reaktion wird bis zu einem Umsatz von > 99 % Richtung Zielprodukt durchgeführt.

### Aufarbeitung ohne weitere Zwischenschritte:

Das Reaktionsgemisch wird unter Rühren und einer Stickstoffatmosphäre auf 20 °C abgekühlt. Das Zielprodukt - der Heterocyclus - fällt sehr feinverteilt aus. Die Filtration dauert ca. 16 Stunden. Auch nach der anschließenden Wäsche mit Methanol ist die Filtrierbarkeit des Zielproduktes extrem schlecht.

Das Beispiel 1 und das Vergleichsbeispiel 1 zeigen, dass in Gegenwart von Aminoalkoholen gemäß Formel (III) keine ausreichende Kristallisation erfolgt und somit ein Abtrennen mittels Filtration so gut wie nicht möglich ist.

### Beispiel 2:

Die Reaktion wird in einem 2 Liter-4-Halsrührkolben mit Innenthermometer, Flügelrührer mit Rührhülse und Rührmotor, Beheizung mittels Ölbad, Wasserauskreiser, Rückflusskühler und einer Abgasleitung mit einem Blasenzähler zur Überwachung des frei werden Ammoniaks durchgeführt. Hierfür werden 600,0 g 3-Amino-1-propanol (7,9 mol) und 5,0 g Zink-2-ethlyhexanoat (0,01 mol) im Reaktionsgefäß vorgelegt und unter Rühren auf eine Temperatur von ca. 135 °C eingestellt. Kontinuierlich werden 128,0 g Terephthalsäuredinitril (1,0 mol) in 2 Stunden zu der Reaktionslösung in den Reaktor gegeben. Nach Ende der Terephthalsäuredinitril-Zugabe wird 5 Stunden bei 135 °C nachgerührt. Im Reaktor liegt eine Lösung vor. Der Reaktionsverlauf wird sowohl über die Abgasbildung als auch über die GC-Analytik bestimmt. Am Ende der Nachreaktionszeit entsteht nur noch minimal Abgas. Die Reaktion wird bis zu einem Umsatz von > 95 % Richtung Zielprodukt durchgeführt.

### Vergleichsbeispiel 2:

Die Reaktion wird in einem 2 Liter-4-Halsrührkolben mit Innenthermometer, Flügelrührer mit Rührhülse und Rührmotor, Beheizung mittels Ölbad, Wasserauskreiser, Rückflusskühler und einer Abgasleitung mit einem Blasenzähler zur Überwachung des frei werden Ammoniaks durchgeführt. Hierfür werden 600,0 g 3-Amino-1-propanol (7,9 mol), 325 g (3 mol) Xylol und 5,0 g Zink-2-ethlyhexanoat (0,01 mol) im Reaktionsgefäß vorgelegt und unter Rühren auf eine Temperatur von ca. 135 °C eingestellt, wobei sich ein leichter Rückfluss eingestellt. Kontinuierlich werden 128,0 g Terephthalsäuredinitril (1,0 mol) in 3 Stunden zu der Reaktionslösung in den Reaktor gegeben. Nach Ende der Terephthalsäuredinitril-Zugabe wird 17 Stunden bei 135 °C nachgerührt. Im Reaktor liegt eine Lösung vor. Der Reaktionsverlauf wird sowohl über die Abgasbildung als auch über die GC-Analytik bestimmt. Am Ende der Nachreaktionszeit entsteht nur noch minimal Abgas. Die Reaktion wird bis zu einem Umsatz von > 95 % Richtung Zielprodukt durchgeführt.

Das Beispiel 2 und das Vergleichsbeispiel 2 zeigen deutlich, dass die Anwesenheit eines zusätzlichen Lösemittels während der Reaktion zu einer deutlichen Verlängerung der Reaktionszeit führt.

## Patentansprüche

1. Verfahren zur Herstellung von Heterocyclen der Formel (I) 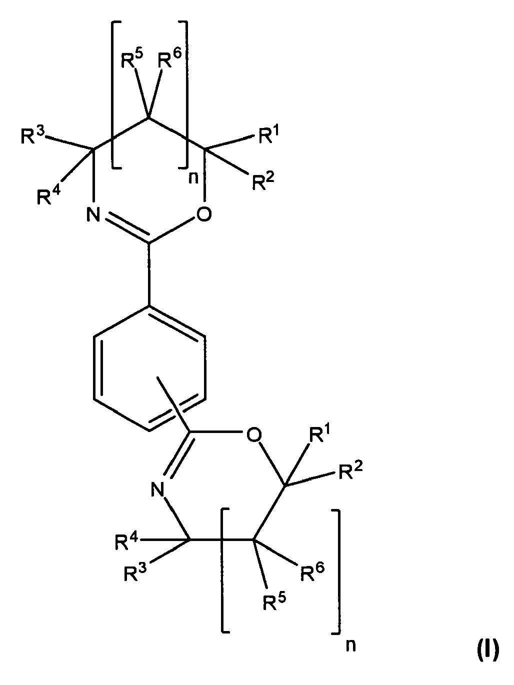 durch katalytische Umsetzung eines aromatischen Dinitrils der Formel (II) 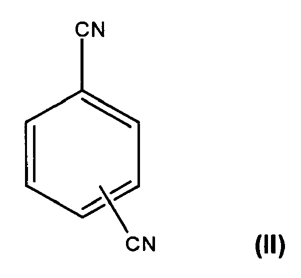 mit einem Aminoalkohol der Formel (III) 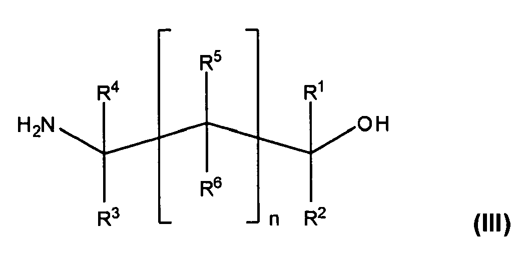 mit R¹, R², R³, R⁴, R⁵ und R⁶ gleich oder voneinander verschieden sind und jeweils Wasserstoff, Alkyl, Aryl, -COOH oder -NH₂ bedeuten und n = 0 bis 4 bedeutet, **dadurch gekennzeichnet,**
**dass** der Aminoalkohol der Formel (III) und der Katalysator vorgelegt und das aromatische Dinitril der Formel (II) bei Reaktionstemperatur zudosiert wird, wobei das molare Verhältnis von Dinitril der Formel (II) zu Aminoalkohol (III) mindestens 1:2 beträgt, wobei auf ein zusätzliches Lösemittel während der Reaktion verzichtet wird, und anschließend nach dem Reaktionsende der überschüssige Aminoalkohol vollständig bzw. nahezu vollständig abgetrennt und durch ein unpolares Lösemittel ersetzt wird, und somit ein Rohprodukt mit einem Gehalt an Aminoalkohol von maximal 20 Gew.-% bezogen auf die Menge an Heterocyclen der Formel (I) erhalten wird.

2. Verfahren gemäß Anspruch 1,
**dadurch gekennzeichnet,**
**dass** ein Aminoalkohol der Formel (III) mit n = 1 bis 4 eingesetzt wird.

3. Verfahren gemäß Anspruch 1 oder 2,
**dadurch gekennzeichnet,**
**dass** Zink-2-ethylhexanoat als Katalysator eingesetzt wird.

4. Verfahren gemäß zumindest einem der Ansprüche 1 bis 3,
**dadurch gekennzeichnet,**
**dass** nach dem Reaktionsende 40 bis 70 Gew.-% des Überschusses an Aminoalkohol der Formel (III) bei einem Druck von 75 bis 400 mbar destillativ entfernt wird und anschließend der in dem Reaktionsgemisch verbleibende Anteil des Aminoalkohols der Formel (III) unter Zugabe eines unpolaren zusätzlichen Lösemittels gemeinsam abdestilliert wird.

5. Verfahren gemäß Anspruch 4,
**dadurch gekennzeichnet,**
**dass** Xylol als zusätzliches unpolares Lösemittel eingesetzt wird.

6. Verfahren gemäß Anspruch 5,
**dadurch gekennzeichnet,**
**dass** ein Rohprodukt mit einem Gehalt an Aminoalkohol von maximal 10 Gew.-% bezogen auf die Menge an Heterocyclen der Formel (I) erhalten wird.

7. Verfahren gemäß Anspruch 6,
**dadurch gekennzeichnet,**
**dass** die Kristalle des Heterocyclen der Formel (I) abfiltriert werden.

8. Verfahren gemäß Anspruch 7,
**dadurch gekennzeichnet,**
**dass** die nach der Filtration zurückbleibende Mutterlauge vor der gemeinsamen Destillation von Aminoalkohol und unpolaren Lösemittel gemeinsam mit oder anstelle des unpolaren Lösemittels in den Verfahrensschritt der Aufarbeitung zurückgeführt wird.

## Claims

1. Process for preparing heterocycles of the formula (I) 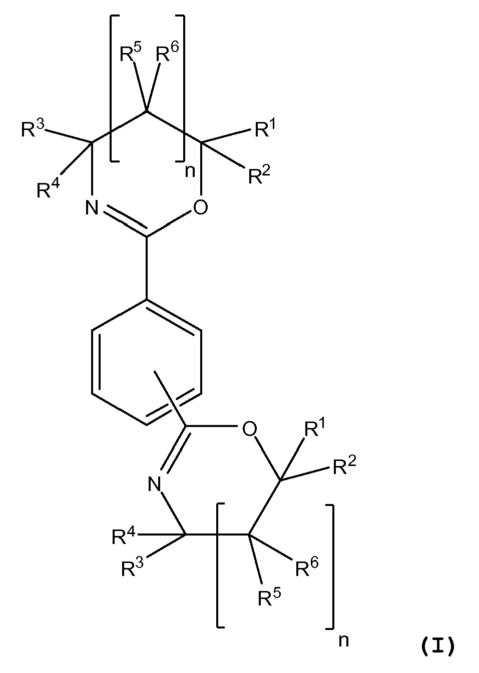 by catalytically reacting an aromatic dinitrile of the formula (II) 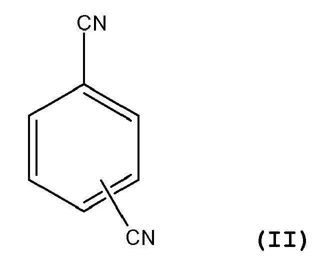 with an amino alcohol of the formula (III) 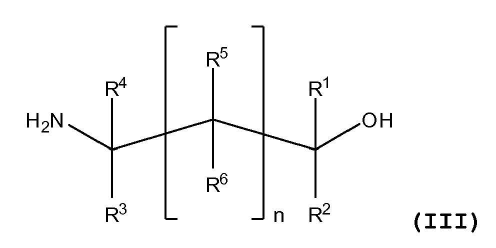 where R¹, R², R³, R⁴, R⁵ and R⁶ are the same or different from one another and are each hydrogen, alkyl, aryl, -COOH or -NH₂ and n = 0 to 4, **characterized in that**
the amino alcohol of the formula (III) and the catalyst are initially charged and the aromatic dinitrile of the formula (II) is metered in at reaction temperature, the molar ratio of dinitrile of the formula (II) to amino alcohol (III) being at least 1:2, an additional solvent being dispensed with during the reaction, and then, after the end of the reaction, the excess amino alcohol is removed completely or virtually completely and is replaced by a nonpolar solvent, and a crude product is thus obtained with an amino alcohol content of not more than 20% by weight, based on the amount of heterocycles of the formula (I).

2. Process according to Claim 1,
**characterized in that**
an amino alcohol of the formula (III) where n = 1 to 4 is used.

3. Process according to Claim 1 or 2,
**characterized in that**
zinc 2-ethylhexanoate is used as the catalyst.

4. A process according to at least one of Claims 1 to 3,
**characterized in that**,
after the end of the reaction, 40 to 70% by weight of the excess of amino alcohol of the formula (III) is removed by distillation at a pressure of 75 to 400 mbar and then the proportion of the amino alcohol of the formula (III) remaining in the reaction mixture is codistilled off with addition of a nonpolar additional solvent.

5. Process according to Claim 4,
**characterized in that**
xylene is used as the additional nonpolar solvent.

6. Process according to Claim 5,
**characterized in that**
a crude product with an amino alcohol content of not more than 10% by weight based on the amount of heterocycles of the formula (I) is obtained.

7. Process according to Claim 6,
**characterized in that**
the crystals of the heterocycle of the formula (I) are filtered off.

8. Process according to Claim 7,
**characterized in that**
the mother liquor which remains after the filtration, before the codistillation of amino alcohol and nonpolar solvent, is recycled into the process step for workup together with or instead of the nonpolar solvent.

## Revendications

1. Procédé pour la préparation d'hétérocycles de formule (I) 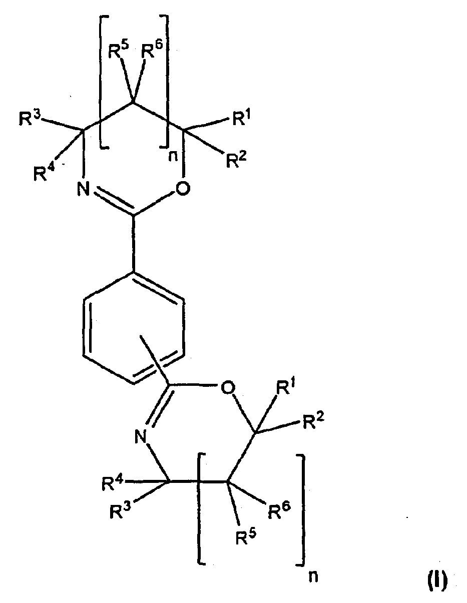 par transformation catalytique d'un dinitrile aromatique de formule (II) 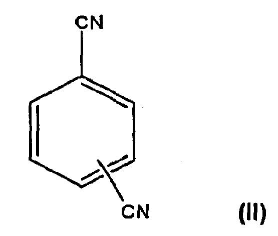 avec un aminoalcool de formule (III) 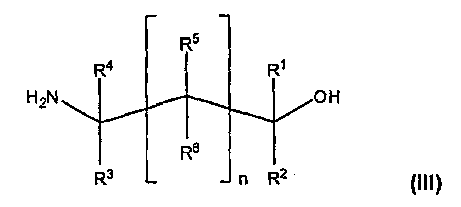 R¹, R², R³, R⁴, R⁵ et R⁶ étant identiques ou différents les uns des autres et signifiant à chaque fois hydrogène, alkyle, aryle, -COOH ou -NH₂ et n = 0 à 4, **caractérisé en ce qu'**on dispose au préalable l'aminoalcool de formule (III) et le catalyseur et on ajoute en dosant le dinitrile aromatique de formule (II) à la température de réaction, le rapport molaire de dinitrile de formule (II) à aminoalcool (III) valant au moins 1:2, en renonçant à un solvant supplémentaire pendant la réaction, puis, après le fin de la réaction, on élimine complètement ou, selon le cas, de manière quasiment complète l'aminoalcool en excès et on le remplace par un solvant non polaire et on obtient donc un produit brut présentant une teneur en aminoalcool d'au maximum 20% en poids par rapport à la quantité d'hétérocycles de formule (I).

2. Procédé selon la revendication 1, **caractérisé en ce qu'**on utilise un aminoalcool de formule (III) avec n = 1 à 4.

3. Procédé selon la revendication 1 ou 2, **caractérisé en ce qu'**on utilise du 2-éthylhexanoate de zinc comme catalyseur.

4. Procédé selon au moins l'une quelconque des revendications 1 à 3, **caractérisé en ce qu'**après la fin de la réaction, 40 à 70% en poids de l'excès d'aminoalcool de formule (III) sont éliminés par distillation à une pression de 75 à 400 mbars, puis la proportion restant dans le mélange réactionnel d'aminoalcool de formule (III) est éliminée conjointement par distillation avec addition d'un solvant non polaire supplémentaire.

5. Procédé selon la revendication 4, **caractérisé en ce qu'**on utilise du xylène comme solvant non polaire supplémentaire.

6. Procédé selon la revendication 5, **caractérisé en ce qu'**on obtient un produit brut présentant une teneur en aminoalcool d'au maximum 10% en poids par rapport à la quantité d'hétérocycles de formule (I).

7. Procédé selon la revendication 6, **caractérisé en ce que** les cristaux d'hétérocycle de formule (I) sont séparés par filtration.

8. Procédé selon la revendication 7, **caractérisé en ce que** la lessive-mère restant après la filtration est recyclée en amont de la distillation conjointe de l'aminoalcool et du solvant non polaire ensemble avec ou au lieu du solvant non polaire dans l'étape de procédé de traitement.
